(19) Europäisches Patentamt
European Patent Office
Office européen des brevets

(11) Numéro de publication : **0 343 053 B1**

# (12) FASCICULE DE BREVET EUROPEEN

(45) Date de publication du fascicule du brevet : **22.01.92 Bulletin 92/04**

(51) Int. Cl.$^5$ : **C07D 327/10**

(21) Numéro de dépôt : **89401335.8**

(22) Date de dépôt : **12.05.89**

## (54) Procédé de préparation de sulfates cycliques.

(30) Priorité : **16.05.88 FR 8806523**

(43) Date de publication de la demande : **23.11.89 Bulletin 89/47**

(45) Mention de la délivrance du brevet : **22.01.92 Bulletin 92/04**

(84) Etats contractants désignés : **AT BE CH DE ES FR GB GR IT LI LU NL SE**

(56) Documents cités :
**DE-B- 1 049 870**
**FR-A- 1 171 347**

(73) Titulaire : **RHONE-POULENC SANTE**
**20, avenue Raymond Aron**
**F-92160 Antony (FR)**

(72) Inventeur : **Massonneau, Viviane**
**Charrière Blanche - Pins 5**
**F-69130 Ecully (FR)**
Inventeur : **Mulhauser, Michel**
**Immeuble "Frênes 4" Résidence Charrière Blanche**
**F-69130 Ecully (FR)**

(74) Mandataire : **Pilard, Jacques et al**
**RHONE-POULENC SANTE, Service Brevets,**
**20 Avenue Raymond Aron**
**F-92165 Antony Cédex (FR)**

EP 0 343 053 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de sulfates cycliques qui sont des intermédiaires utilisables en chimie organique pour réaliser en particulier des réactions d'hydroxyéthylation.

D'après le brevet allemand DE 1049870, il est connu de préparer le sulfate d'éthylène par chauffage au reflux pendant 50 heures d'un mélange d'éthylèneglycol, d'acide sulfurique et d'un excès de chlorure de thionyle.

Il est connu également, d'après J. Lichtenberger et R. Lichtenberger, Bull. Soc. Chim. (France), 1002 (1948) de préparer des sulfates cycliques de diols par action d'un oléum sur une solution chloroformique du diol en mettant en jeu au moins deux moles de $SO_3$ libre par mole de diol, l'oléum ayant une concentration de 47% en $SO_3$. Cependant si cette méthode convient pour les diols primaire-secondaire, elle est inopérante dans le cas de l'éthylène-glycol.

Il a maintenant été trouvé, et c'est ce qui fait l'objet de la présente invention que les sulfates cycliques de formule générale :

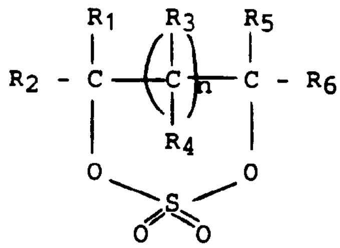

(I)

dans laquelle les substituants $R_1$, $R_2$, $R_3$, $R_4$, $R_5$ et $R_6$, identiques ou différents, représentent un atome d'hydrogène ou d'halogène, un radical alkyle, aryle, alkoxy, aryloxy ou alkoxycarbonyle et n est égal à 0 ou 1, étant entendu que les radicaux alkyles et les portions alkyles des radicaux alkoxy ou alkoxycarbonyles contiennent 1 à 4 atomes de carbone et peuvent être éventuellement substitués par un ou plusieurs atomes ou radicaux identiques ou différents choisis parmi les atomes d'halogène et les radicaux alkoxy, aryloxy ou alkoxycarbonyles, et que les radicaux aryles et les portions aryles des radicaux aryloxy contiennent 6 à 10 atomes de carbone et peuvent être éventuellement substitués par un ou plusieurs atomes ou radicaux identiques ou différents choisis parmi les atomes d'halogène et les radicaux alkyles, alkoxy, aryloxy ou alkoxycarbonyles, peuvent être obtenus par un procédé simple et peu coûteux qui consiste à faire réagir rapidement à une température comprise entre 150 et 250°C l'acide sulfurique concentré avec un glycol de formule générale :

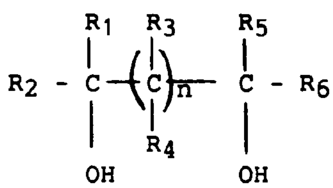

(II)

dans laquelle $R_1$, $R_2$, $R_3$, $R_4$, $R_5$, $R_5$ et n sont définis comme précédemment.

Le sulfate cyclique de formule générale (I) est piégé par refroidissement ou par solubilisation dans un solvant convenable.

Généralement, on utilise un mélange sensiblement équimoléculaire de glycol de formule générale (II) et d'acide sulfurique concentré.

De préférence, le procédé est mis en oeuvre sous une pression réduite, généralement voisine de 1 mm de mercure (0,13 kPa).

Par exemple, le procédé peut être mis en oeuvre en faisant passer rapidement le mélange d'acide sulfurique concentré et de glycol de formule générale (II) dans un réacteur tubulaire chauffé à une température comprise entre 150 et 250°C et maintenu sous pression réduite.

Il peut être avantageux de véhiculer le mélange des réactifs par un gaz vecteur inerte.

Plus particulièrement, la présente invention concerne la préparation des sulfates cycliques de formule générale (I) dans laquelle n est égal à 0 ou 1 et les symboles $R_1$, $R_2$, $R_3$, $R_4$, $R_5$ et $R_5$, identiques ou différents représentent un atome d'hydrogène ou un radical alkyle.

L' exemple suivant, donné à titre non limitatif, montre comment l'invention peut être mise en pratique.

EXEMPLE

On mélange 6,2 g d'éthylèneglycol (0,1 mole) et 9,8 g d'acide sulfurique à 97% (0,097 mole).

On introduit, à l'aide d'une seringue, 2,5 g du mélange dans le réacteur représenté par la figure 1. Le réacteur est chauffé à 200°C par un ruban chauffant. L'appareillage est placé sous une pression réduite de 1 mm de mercure (0,13 kPa). Le temps de séjour du mélange éthylène glycol — acide sulfurique dans la partie chauffée du réacteur est de 10 minutes.

Le sulfate d'éthylène formé est recueilli dans la colonne Vigreux placée dans un manchon de carboglace. Après la fin de la réaction, la colonne Vigreux est rincée au dichlorométhane. Après décantation de la solution obtenue et évaporation du dichlorométhane, on obtient 0,85 g de sulfate d'éthylène sous forme de cristaux blancs. Le rendement est de 45% par rapport à l'acide sulfurique mis en oeuvre.

Dans la figure 1 :

— (1) représente l'orifice d'introduction des réactifs
— (2) représente un tube de coulée des réactifs
— (3) représente un ruban chauffant
— (4) représente le récipient de réception du sulfate d'éthylène après rinçage du Vigreux
— (5) représente une colonne Vigreux
— (6) représente un manchon réfrigérant
— (7) représente la prise de mise sous pression réduite.

## Revendications

1. Procédé de préparation d'un sulfate cyclique de formule générale :

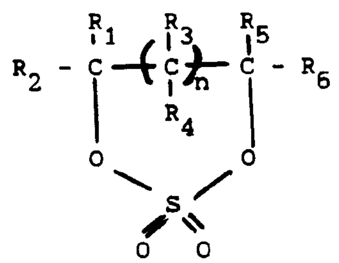

dans laquelle $R_1$, $R_2$, $R_3$, $R_4$, $R_5$ et $R_6$, identiques ou différents, représentent un atome d'hydrogène ou d'halogène ou un radical alkyle, aryle, alkoxy, aryloxy ou alkoxycarbonyle et n est égal à 0 ou 1, étant entendu que les radicaux alkyles et les portions alkyles des radicaux alkoxy ou alkoxycarbonyles contiennent 1 à 4 atomes de carbone et sont éventuellement substitués par un ou plusieurs atomes ou radicaux, identiques ou différents choisis parmi les atomes d'halogène et les radicaux alkoxy, aryloxy ou alkoxycarbonyles, et que les radicaux aryles et les radicaux aryloxy contiennent 6 à 10 atomes de carbone et sont éventuellement substitués par un ou plusieurs atomes ou radicaux, identiques ou différents choisis parmi les atomes d'halogène et les radicaux alkyles, alkoxy, aryloxy ou alcoxycarbonyles, et n est égal à 0 ou 1, caractérisé en ce que l'on fait réagir rapidement à une température comprise entre 150 et 250°C l'acide sulfurique concentré avec un glycol de formule générale :

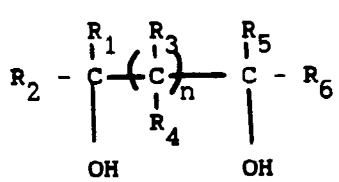

dans laquelle $R_1$, $R_2$, $R_3$, $R_4$, $R_5$, $R_6$ et n sont définis comme précédemment et récupère le sulfate cyclique obtenu.

2. Procédé de préparation d'un sulfate cyclique de formule générale :

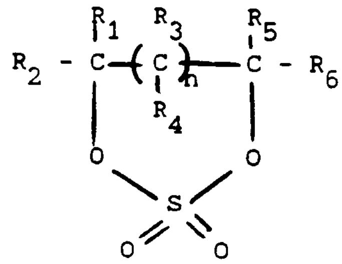

dans laquelle $R_1$, $R_2$, $R_3$, $R_4$, $R_5$ et $R_5$, identiques ou différents, représentent un atome d'hydrogène ou un un radical alkyle et n est égal à 0 ou 1 caractérisé en ce que l'on fait réagir rapidement à une température comprise entre 150 et 250°C l'acide sulfurique concentré avec un glycol de formule générale :

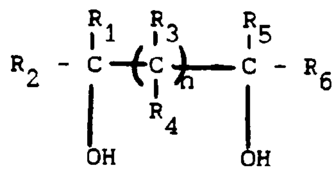

dans laquelle $R_1$, $R_2$, $R_3$, $R_4$, $R_5$, $R_6$ et n sont définis comme précédemment et récupère le sulfate cyclique obtenu.

3. Procédé selon l'une des revendications 1 ou 2 caractérisé en ce que l'on utilise un mélange sensiblement équimoléculaire de glycol et d'acide sulfurique concentré.

4. Procédé selon l'une des revendications 1, 2 ou 3 caractérisé en ce que l'on opère sous pression réduite.

5. Procédé selon la revendication 4 caractérisé en ce que la pression est voisine de 1 mm de mercure ou 0,13 kPa.

6. Procédé selon la revendication 1 caractérisé en ce que le mélange de glycol et d'acide sulfurique est véhiculé par un gaz inerte.

## Claims

1. Process for the preparation of a cyclic sulphate of general formula :

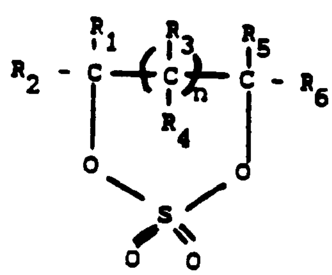

in which $R_1$, $R_2$, $R_3$, $R_4$, $R_5$ and $R_6$, which are identical or different, denote a hydrogen or halogen atom or an alkyl, aryl, alkoxy, aryloxy or alkoxycarbonyl radical and n is equal to 0 or 1, it being understood that the alkyl radicals and the alkyl moieties of the alkoxy or alkoxycarbonyl radicals contain 1 to 4 carbon atoms and are optionally substituted by one or more identical or different atoms or radicals chosen from halogen atoms and alkoxy, aryloxy or alkoxycarbonyl radicals, and that the aryl radicals and the aryloxy radicals contain 6 to 10 carbon atoms and are optionally substituted by one or more identical or different atoms or radicals chosen from halogen atoms and alkyl, alkoxy, aryloxy or alkoxycarbonyl radicals, and n is equal to 0 or 1, characterised in that concentrated sulphuric acid is rapidly reacted, at a temperature of between 150 and 250°C, with a glycol

of general formula :

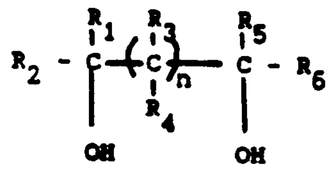

in which $R_1$, $R_2$, $R_3$, $R_4$, $R_5$, $R_6$ and n are defined as above and the cyclic sulphate obtained is recovered.

2. Process for the preparation of a cyclic sulphate of general formula :

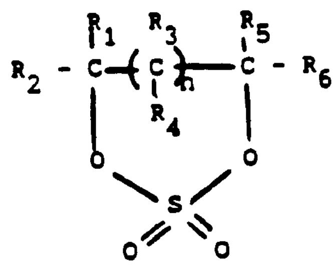

in which $R_1$, $R_2$, $R_3$, $R_4$, $R_5$ and $R_6$, which are identical or different, denote a hydrogen atom or an alkyl radical and n is equal to 0 or 1, characterised in that concentrated sulphuric acid is rapidly reacted, at a temperature of between 150 and 250°C, with a glycol of general formula :

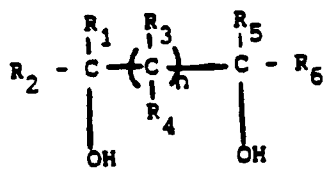

in which $R_1$, $R_2$, $R_3$, $R_4$, $R_5$, $R_6$ and n are defined as above and the cyclic sulphate obtained is recovered.

3. Process according to either of Claims 1 and 2, characterised in that a substantially equimolar mixture of glycol and of concentrated sulphuric acid is employed.

4. Process according to one of Claims 1, 2 and 3, characterised in that the operation is carried out under reduced pressure.

5. Process according to Claim 4, characterised in that the pressure is close to 1 mm Hg or 0.13 kPa.

6. Process according to Claim 1, characterised in that the mixture of glycol and of sulphuric acid is conveyed by an inert gas.

**Patentansprüche**

1. Verfahren zur Herstellung eines cyclischen Sulfats der allgemeinen Formel :

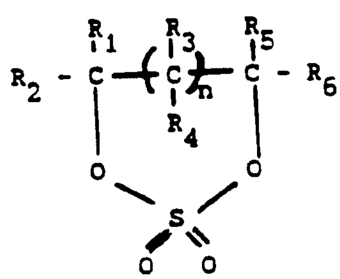

in welcher $R_1$, $R_2$, $R_3$, $R_4$, $R_5$ und $R_5$ unabhängig voneinander ein Wasserstoff- oder Halogenatom oder einen

Alkyl-, Aryl-, Alkoxy-, Aryloxy- oder Alkoxycarbonylrest darstellen und n gleich 0 oder 1 ist, wobei die Alkylreste und Alkylteile der Alkoxy- oder Alkoxycarbonylreste selbstverständlich 1 bis 4 Kohlenstoffatome enthalten und gegebenenfalls durch ein oder mehrere gleiche oder voneinander verschiedene Atome oder Reste, ausgewählt aus den Halogenatomen und den Alkoxy-, Aryloxy- oder Alkoxycarbonylresten, substituiert sind, und die Arylreste und die Aryloxyreste 6 bis 10 Kohlenstoffatome enthalten und gegebenenfalls durch ein oder mehrere gleiche oder voneinander verschiedene Atome oder Reste, ausgewählt aus den Halogenatomen und den Alkyl-, Alkoxy-, Aryloxy- oder Alkoxycarbonylresten, substituiert sind, und n gleich 0 oder 1 ist, dadurch gekennzeichnet, daß man konzentrierte Schwefelsäure mit einem Glykol der allgemeinen Formel :

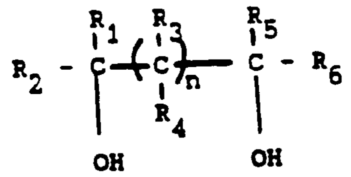

in welcher $R_1$, $R_2$, $R_3$, $R_4$, $R_5$, $R_6$ und n die obige Bedeutung haben, rasch bei einer Temperatur zwischen 150 und 250°C reagieren läßt und das erhaltene cyclische Sulfat gewinnt.

2. Verfahren zur Herstellung eines cyclischen Sulfats der allgemeinen Formel :

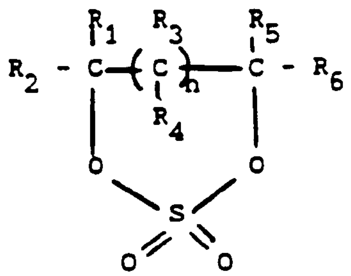

in welcher $R_1$, $R_2$, $R_3$, $R_4$, $R_6$ und $R_6$ unabhängig voneinander ein Wasserstoff- oder Halogenatom oder einen Alkylrest darstellen und n gleich 0 oder 1 ist, dadurch gekennzeichnet, daß man konzentrierte Schwefelsäure mit einem Glykol der allgemeinen Formel :

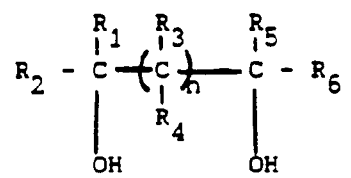

in welcher $R_1$, $R_2$, $R_3$, $R_4$, $R_5$, $R_6$ und n die obige Bedeutung haben, rasch bei einer Temperatur zwischen 150 und 250°C reagieren läßt und das erhaltene cyclische Sulfat gewinnt.

3. Verfahren nach einem der Ansprüche 1 oder 2, dadurch gekennzeichnet, daß man eine im wesentlichen äquimolare Mischung von Glykol und konz. Schwefelsäure verwendet.

4. Verfahren nach einem der Ansprüche 1, 2 oder 3, dadurch gekennzeichnet, daß man unter vermindertem Druck arbeitet.

5. Verfahren nach Anspruch 4, dadurch gekennzeichnet, daß der Druck nahe 1 mm Hg oder 0,13 kPa ist.

6. Verfahren nach Anspruch 1, dadurch gekennzeichnet, daß die Mischung von Glykol und Schwefelsäure durch ein Inertgas transportiert wird.

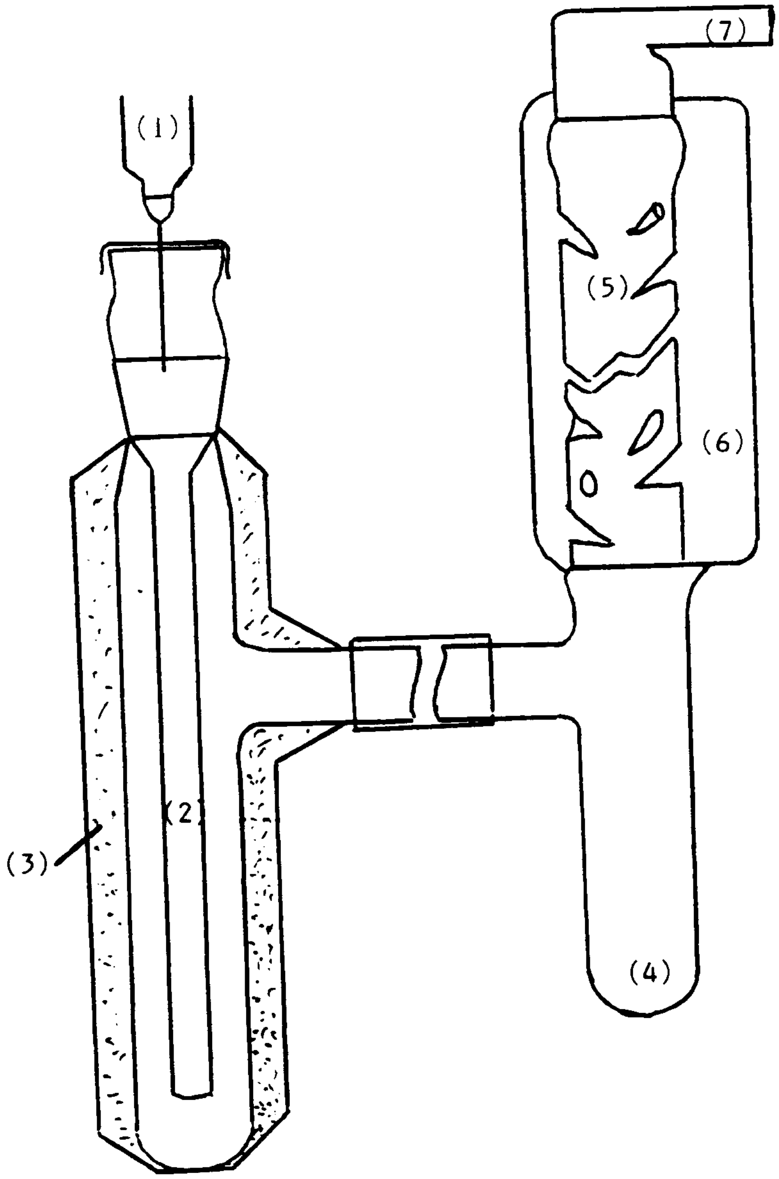


FIGURE 1